# EUROPEAN PATENT APPLICATION

(11) **EP 4 591 857 A1**
(43) Date of publication of application: **30.07.2025**
(21) Application number: 23867503.7
(22) Date of filing: 19.09.2023
(51) Int. Cl.: A61K 9/16, A61K 38/26, A61K 9/50, A61K 9/52, A61K 47/34, A61P 3/10

(54) **MICROSPHERE**

(30) Priority: 19.09.2022 CN 202211138917
(71) Applicant: Bostal Drug Delivery Co., Ltd., Guangzhou, Guangdong 510320 (CN)
(72) Inventor: LIU, Rong, Guangzhou, Guangdong 510320 (CN); GUI, Chunhua, Guangzhou, Guangdong 510320 (CN); LI, Wei, Guangzhou, Guangdong 510320 (CN); TAO, Hui, Guangzhou, Guangdong 510320 (CN); WAN, Xiaoli, Guangzhou, Guangdong 510320 (CN)
(74) Representative: Müller-Boré & Partner Patentanwälte PartG mbB
(86) International application number: PCT/CN2023/119727
(87) International publication number: WO 2024/061216

(57) **Abstract**

Disclosed are microspheres comprising semaglutide and a pharmaceutically acceptable polymer, wherein drug loading of the microspheres is from 11.0% to 20.0% and 90% of cumulative pore volume of internal voids of the microspheres is about from 0.013 to 0.20 mL/g.

## Description

### REFERENCE TO RELATED APPLICATION

The present disclosure claims all the benefits of the Chinese patent application No. 202211138917.9, entitled Microspheres, filed with the China Intellectual Property Office of the People's Republic of China on September 19, 2022, which is incorporated herein by reference in its entirety.

### FIELD

The present disclosure relates generally to the field of biology and medicine, and more particularly, to the field of pharmaceutical formulations.

### BACKGROUND

Glucagon-like peptide-1 (GLP-1) is a hormone produced primarily by intestinal L cells and belongs to incretin.

### SUMMARY

In one aspect, the present disclosure relates to microspheres, comprising semaglutide and a pharmaceutically acceptable polymer, wherein drug loading of the microspheres is from 11.0% to 12.5% and 90% of cumulative pore volume of internal voids of the microspheres is about 0.013 to 0.15 mL/g.

In another aspect, the present disclosure relates to microspheres, comprising semaglutide and a pharmaceutically acceptable polymer, wherein drug loading of the microspheres is from 11.0% to 20.0% and 90% of cumulative pore volume of internal voids of the microspheres is about 0.013 to 0.20mL/g.

In yet another aspect, the present disclosure relates to a pharmaceutical composition comprising microspheres, wherein the microspheres comprise semaglutide and a pharmaceutically acceptable polymer, wherein drug loading of the microspheres is from 11.0% to 12.5% and 90% of cumulative pore volume of internal voids of the microspheres is about 0.013 to 0.15 mL/g.

In yet another aspect, the present disclosure relates to a pharmaceutical composition comprising microspheres, wherein the microspheres comprise semaglutide and a pharmaceutically acceptable polymer, wherein drug loading of the microspheres is from 11.0% to 20.0% and 90% of cumulative pore volume of internal voids of the microspheres is about 0.013 to 0.20 mL/g.

In another aspect, the disclosure relates to a process for preparing microspheres, comprising: providing a first aqueous solution comprising semaglutide; providing an oil solution comprising a pharmaceutically acceptable polymer; emulsifying the first aqueous solution and the oil solution to obtain a water-in-oil (W/O) emulsion; mixing the water-in-oil (W/O) emulsion with a second aqueous solution to obtain a W/O/W emulsion; and removing solvent in oil phase of the W/O/W emulsion to obtain the microspheres, wherein drug loading amount of the microspheres is from 11.0% to 12.5% and 90% of cumulative pore volume of internal voids of the microspheres is about 0.013 to 0.15 mL/g.

In yet another aspect, the present disclosure relates to a process for preparing microspheres, comprising: providing a first aqueous solution comprising semaglutide; providing an oil solution comprising a pharmaceutically acceptable polymer; emulsifying the first aqueous solution and the oil solution to obtain a water-in-oil (W/O) emulsion; mixing the water-in-oil (W/O) emulsion with a second aqueous solution to obtain a water-in-oil-in-water (W/O/W) emulsion; and removing solvent in oil phase of the W/O/W emulsion to obtain the microspheres, wherein drug loading amount of the microspheres is from 11.0% to 20.0% and 90% of cumulative pore volume of internal voids of the microspheres is about 0.013 to 0.20 mL/g.

In a further aspect, the present disclosure relates to microspheres prepared by a process comprising: providing a first aqueous solution comprising semaglutide; providing an oil solution containing a pharmaceutically acceptable polymer; emulsifying the first aqueous solution and the oil solution to obtain a water-in-oil (W/O) emulsion; mixing the water-in-oil (W/O) emulsion with a second aqueous solution to obtain a water-in-oil-in-water (W/O/W) emulsion; and removing solvent in oil phase of the W/O/W emulsion to obtain the microspheres, wherein drug loading amount of the microspheres is from 11.0% to 12.5% and 90% of cumulative pore volume of internal voids of the microspheres is about 0.013 to 0.15 mL/g.

In another aspect, the present disclosure relates to microspheres prepared by a process comprising: providing a first aqueous solution comprising semaglutide; providing an oil solution containing a pharmaceutically acceptable polymer; emulsifying the first aqueous solution and the oil solution to obtain a water-in-oil (W/O) emulsion; mixing the water-in-oil (W/O) emulsion with a second aqueous solution to obtain a water-in-oil-in-water (W/O/W) emulsion; and removing solvent in oil phase of the W/O/W emulsion to obtain the microspheres, wherein drug loading amount of the microspheres is from 11.0% to 20.0% and 90% of cumulative pore volume of internal voids of the microspheres is about 0.013 to 0.20 mL/g.

In yet another aspect, the present disclosure relates to a method for treating diabetes, comprising administering to a subject in need thereof a therapeutically effective amount of the microspheres described in the present disclosure, the pharmaceutical composition described in the present disclosure, or the microspheres prepared by the process described in the present disclosure.

In a further aspect, the present disclosure relates to a method for reducing body weight, comprising administering to a subject in need thereof an effective amount of the microspheres described in the present disclosure, the pharmaceutical composition described in the present disclosure, or the microspheres prepared by the process described in the present disclosure.

### BRIEF DESCRIPTION OF THE DRAWINGS

Fig.1 shows pore volume/pore size distribution curves of the microspheres of Example 1;
Fig.2 shows pore volume/pore size distribution curves of the microspheres of Example 2;
Fig.3 shows pore volume/pore size distribution curves of the microspheres of Example 3;
FIG. 4 shows pore volume/pore size distribution curves of the microspheres of Example 4;
Fig.5 shows pore volume/pore size distribution curves of the microspheres of Example 5;
Fig. 6 shows the *in vitro* release profile of the microspheres of Example 1;
Fig. 7 shows the *in vitro* release profile of the microspheres of Example 2;
Fig. 8 shows the *in vitro* release profile of the microspheres of Example 3;
Fig. 9 shows the *in vitro* release profile of the microspheres of Example 4;
Fig. 10 shows the *in vitro* release profile of the microspheres of Example 5;
Fig. 11 shows the *in vitro* release profile of the microspheres of Example 6;
Fig. 12 shows the drug concentration versus time profile of the microspheres of Example 1;
Fig. 13 shows the drug concentration versus time profile of the microspheres of Example 2;
Fig. 14 shows the drug concentration versus time profile of the microspheres of Example 3;
Fig. 15 shows the drug concentration versus time profile of the microspheres of Example 4; and
Fig. 16 shows the drug concentration versus time profile of the microspheres of Example 5.

### DETAILED DESCRIPTION

In the following description, certain specific details are included to provide a thorough understanding for various disclosed embodiments. One skilled in the relevant art, however, will recognize that the embodiments may be practiced without one or more these specific details, or with other methods, components, materials, etc.

Unless the context required otherwise, throughout the specification and claims which follows, the term "comprise" and variations thereof, such as "comprises" and "comprising" are to be construed in an open, inclusive sense, which is as "include, but not limited to".

Reference throughout this specification to "one embodiment", or "an embodiment", or "in another embodiment", or "in some embodiments" means that a particular referent feature, structure or characteristic described in connection with the embodiments is included in at least one embodiment. Therefore, the appearance of the phrases "in one embodiment", or "in the embodiment", or "in another embodiment", or "in some embodiments" in various places throughout this specification are not necessarily all referring to the same embodiment. Moreover, the particular features, structures or characteristics may be combined in any suitable manner in one or more embodiments.

### Definition

In the present disclosure, the term "pore volume" refers to the volume of internal voids per mass of a porous solid.

In the present disclosure, the term "cumulative pore volume" refers to the cumulative volume of internal voids per mass of a porous solid.

In the present disclosure, the term "semaglutide", refers to N^{ε26}[(S)-(22,40-dicarboxylic acid-10,19,24-trioxo-3,6,12,15-tetraoxa-9,18,23-triazatetradecane-1-acyl)][Aib⁸,Arg³⁴ ]GLP-1- (7-37) peptide, which has the following structural formula:

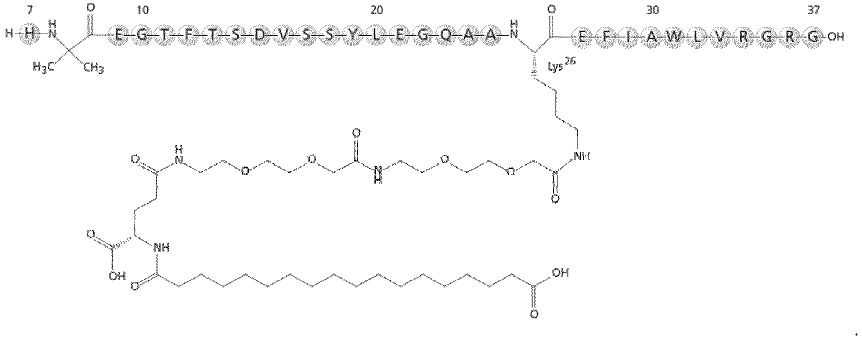

In the present disclosure, the term "bioavailability" refers to the relative amount of drug that reaches the blood circulation from the administration site.

In the present disclosure, the term "AUC_{inf}" refers to the area under the drug concentration-time curve extrapolated to infinite time in the drug concentration-time curve of the formulation.

In the present disclosure, the term "drug loading" refers to the weight percent of the drug contained in the particulate formulation, i.e., drug loading = the weight of the drug contained in the particulate formulation/the total weight of the particulate formulation ×100%.

In the present disclosure, the term "poly(D,L-lactide-co-glycolide) polymer (PLGA)" is also referred to as D,L-lactic and glycolic acids copolymer, formed by condensation polymerization of two monomers, lactic acid and glycolic acid, or ring-opening polymerization of lactide and glycolide.

### Specific Embodiments

In one aspect, the present disclosure relates to microspheres, comprising semaglutide and a pharmaceutically acceptable polymer, wherein drug loading of the microspheres is about 11.0% to 12.5% and 90% of cumulative pore volume of internal voids of the microspheres is about 0.013 to 0.15 mL/g.

In another aspect, the present disclosure relates to microspheres, comprising semaglutide and a pharmaceutically acceptable polymer, wherein drug loading of the microspheres is from 11.0% to 20.0% and 90% of cumulative pore volume of internal voids of the microspheres is about 0.013 to 0.20mL/g.

In some embodiments, the drug loading of the microspheres of the present disclosure is about 11.1%.

In some embodiments, the drug loading of the microspheres of the present disclosure is about 11.2%.

In some embodiments, the drug loading of the microspheres of the present disclosure is about 11.3%.

In some embodiments, the drug loading of the microspheres of the present disclosure is about 11.4%.

In some embodiments, the drug loading of the microspheres of the present disclosure is about 11.5%.

In some embodiments, the drug loading of the microspheres of the present disclosure is about 11.6%.

In some embodiments, the drug loading of the microspheres of the present disclosure is about 11.7%.

In some embodiments, the drug loading of the microspheres of the present disclosure is about 11.8%.

In some embodiments, the drug loading of the microspheres of the present disclosure is about 11.9%.

In some embodiments, the drug loading of the microspheres of the present disclosure is about 12.0%.

In some embodiments, the drug loading of the microspheres of the present disclosure is about 12.1%.

In some embodiments, the drug loading of the microspheres of the present disclosure is about 12.2%.

In some embodiments, the drug loading of the microspheres of the present disclosure is about 12.3%.

In some embodiments, the drug loading of the microspheres of the present disclosure is about 12.4%.

In some embodiments, the microspheres of the present disclosure have a drug loading of about 12.5%, 12.6%, 12.7%, 12.8%, 12.9%, 13.0%, 13.1%, 13.2%, 13.3%, 13.4%, 13.5%, 13.6%, 13.7%, 13.8%, 13.9%, 14.0%, 14.1%, 14.2%, 14.3%, 14.4%, 14.5%, 14.6%, 14.7%, 14.8%, 14.9%, 15.0%, 15.1%, 15.2%, 15.3%, 15.4%, 15.5%, 15.6%, 15.7%, 15.8%, 15.9%, 16.0%, 16.1%, 16.2%, 16.3%, 16.4%, 16.5%, 16.6%, 16.7%, 16.8%, 16.9%, 17.0%, 17.1%, 17.2%, 17.3%, 17.4%, 17.5%, 17.6%, 17.7%, 17.8%, 17.9%, 18.0%, 18.1%, 18.2%, 18.3%, 18.4%, 18.5%, 18.6%, 18.7%, 18.8%, 18.9%, 19.0%, 19.1%, 19.2%, 19.3%, 19.4%, 19.5%, 19.6%, 19.7%, 19.8%, 19.9%, or 20.0%.

In some embodiments, the drug loading of the microspheres of the present disclosure is about 11.0% to 19.0%, or 11.0% to 18.0%.

In some embodiments, 90% of the cumulative pore volume of the internal voids of the microspheres of the present disclosure is about 0.017 to 0.12 mL/g.

In some embodiments, 90% of the cumulative pore volume of the internal voids of the microspheres of the present disclosure is about 0.014 to 0.19mL/g, 0.015 to 0.18mL/g, 0.016 to 0.17mL/g, 0.017 to 0.18mL/g, 0.017 to 0.17mL/g, 0.017 to 0.16mL/g, or 0.017 to 0.15 mL/g.

In some embodiments, 50% of the cumulative pore volume of the internal voids of the microspheres of the present disclosure is about 0.013 to 0.079 mL/g.

In some embodiments, 50% of the cumulative pore volume of the internal voids of the microspheres of the present disclosure is about 0.016 to 0.066 mL/g.

In some embodiments, 50% of the cumulative pore volume of the internal voids of the microspheres of the present disclosure is about 0.013 to 0.11 mL/g, 0.014 to 0.10 mL/g, 0.015 to 0.09 mL/g, or 0.016 to 0.08 mL/g.

In some embodiments, 10% of the cumulative pore volume of the internal voids of the microspheres of the present disclosure is about 0.009 to 0.016 mL/g.

In some embodiments, 10% of the cumulative pore volume of the internal voids of the microspheres of the present disclosure is about 0.011 to 0.014 mL/g.

In some embodiments, 10% of the cumulative pore volume of the internal voids of the microspheres of the present disclosure is about 0.011 to 0.022 mL/g, 0.011 to 0.021 mL/g, 0.011 to 0.020 mL/g, 0.011 to 0.019 mL/g, 0.011 to 0.018 mL/g, 0.011 to 0.017 mL/g, or 0.011 to 0.016 mL/g.

In some embodiments, exemplary examples of pharmaceutically acceptable polymers that can be used in the present disclosure include, but are not limited to, poly(D,L-lactide-co-glycolide) polymer (PLGA).

In some embodiments, the content of poly(D,L-lactide-co-glycolide) polymer (PLGA) in the microspheres is about 80% to 85% by weight based on the weight of the microspheres.

In some embodiments, the content of the poly(D,L-lactide-co-glycolide) copolymer (PLGA) in the microspheres is about 75% to 85% by weight, 76% to 85% by weight, 77% to 85% by weight, or 78% to 85% by weight, based on the weight of the microspheres.

In some embodiments, the microspheres of the present disclosure may also comprise sucrose.

In some embodiments, the content of sucrose in the microspheres is about 1% to 3% by weight, based on the weight of the microspheres.

In some embodiments, the amount of sucrose in the microspheres is about 2% by weight based on the weight of the microspheres.

In some embodiments, the microspheres of the present disclosure have improved bioavailability.

In some embodiments, the release period of semaglutide from the microspheres of the present disclosure is about 2 weeks or more.

In some embodiments, semaglutide is sustainedly released from microspheres of the present disclosure.

In another aspect, the present disclosure relates to a pharmaceutical composition comprising microspheres, wherein the microspheres comprise semaglutide and a pharmaceutically acceptable polymer, wherein drug loading of the microspheres is from 11.0% to 12.5% and 90% of cumulative pore volume of internal voids of the microspheres is about 0.013 to 0.15 mL/g.

In yet another aspect, the present disclosure relates to a pharmaceutical composition comprising microspheres, wherein the microspheres comprise semaglutide and a pharmaceutically acceptable polymer, wherein drug loading of the microspheres is from 11.0% to 20.0% and 90% of cumulative pore volume of internal voids of the microspheres is about 0.013 to 0.20 mL/g.

In a further aspect, the present disclosure relates to a process for preparing microspheres, comprising: providing a first aqueous solution comprising semaglutide; providing an oil solution containing a pharmaceutically acceptable polymer; emulsifying the first aqueous solution and the oil solution to obtain a water-in-oil (W/O) emulsion; mixing the water-in-oil (W/O) emulsion with a second aqueous solution to obtain a water-in-oil-in-water (W/O/W) emulsion; and removing solvent in oil phase of the W/O/W emulsion to obtain the microspheres, wherein drug loading amount of the microspheres is from 11.0% to 12.5% and 90% of cumulative pore volume of internal voids of the microspheres is about 0.013 to 0.15 mL/g.

In another aspect, the disclosure relates to a process for preparing microspheres, comprising: providing a first aqueous solution comprising semaglutide; providing an oil solution containing a pharmaceutically acceptable polymer; emulsifying the first aqueous solution and the oil solution to obtain a water-in-oil (W/O) emulsion; mixing the water-in-oil (W/O) emulsion with a second aqueous solution to obtain a W/O/W emulsion; and removing solvent in oil phase of the W/O/W emulsion to obtain the microspheres, wherein drug loading amount of the microspheres is from 11.0% to 20.0%, and 90% of cumulative pore volume of internal voids of the microspheres is about 0.013 to 0.20 mL/g.

In some embodiments, the drug loading of the microspheres of the present disclosure is about 11.1%.

In some embodiments, the drug loading of the microspheres of the present disclosure is about 11.2%.

In some embodiments, the drug loading of the microspheres of the present disclosure is about 11.3%.

In some embodiments, the drug loading of the microspheres of the present disclosure is about 11.4%.

In some embodiments, the drug loading of the microspheres of the present disclosure is about 11.5%.

In some embodiments, the drug loading of the microspheres of the present disclosure is about 11.6%.

In some embodiments, the drug loading of the microspheres of the present disclosure is about 11.7%.

In some embodiments, the drug loading of the microspheres of the present disclosure is about 11.8%.

In some embodiments, the drug loading of the microspheres of the present disclosure is about 11.9%.

In some embodiments, the drug loading of the microspheres of the present disclosure is about 12.0%.

In some embodiments, the drug loading of the microspheres of the present disclosure is about 12.1%.

In some embodiments, the drug loading of the microspheres of the present disclosure is about 12.2%.

In some embodiments, the drug loading of the microspheres of the present disclosure is about 12.3%.

In some embodiments, the drug loading of the microspheres of the present disclosure is about 12.4%.

In some embodiments, the microspheres of the present disclosure have a drug loading of about 12.5%, 12.6%, 12.7%, 12.8%, 12.9%, 13.0%, 13.1%, 13.2%, 13.3%, 13.4%, 13.5%, 13.6%, 13.7%, 13.8%, 13.9%, 14.0%, 14.1%, 14.2%, 14.3%, 14.4%, 14.5%, 14.6%, 14.7%, 14.8%, 14.9%, 15.0%, 15.1%, 15.2%, 15.3%, 15.4%, 15.5%, 15.6%, 15.7%, 15.8%, 15.9%, 16.0%, 16.1%, 16.2%, 16.3%, 16.4%, 16.5%, 16.6%, 16.7%, 16.8%, 16.9%, 17.0%, 17.1%, 17.2%, 17.3%, 17.4%, 17.5%, 17.6%, 17.7%, 17.8%, 17.9%, 18.0%, 18.1%, 18.2%, 18.3%, 18.4%, 18.5%, 18.6%, 18.7%, 18.8%, 18.9%, 19.0%, 19.1%, 19.2%, 19.3%, 19.4%, 19.5%, 19.6%, 19.7%, 19.8%, 19.9%, or 20.0%.

In some embodiments, the drug loading of the microspheres of the present disclosure is about 11.0% to 19.0%, or 11.0% to 18.0%.

In some embodiments, 90% of the cumulative pore volume of the internal voids of the microspheres of the present disclosure is about 0.017 to 0.12 mL/g.

In some embodiments, 90% of the cumulative pore volume of the internal voids of the microspheres of the present disclosure is about 0.014 to 0.19mL/g, 0.015 to 0.18mL/g, 0.016 to 0.17mL/g, 0.017 to 0.18mL/g, 0.017 to 0.17mL/g, 0.017 to 0.16mL/g, or 0.017 to 0.15 mL/g.

In some embodiments, 50% of the cumulative pore volume of the internal voids of the microspheres of the present disclosure is about 0.013 to 0.079 mL/g.

In some embodiments, 50% of the cumulative pore volume of the internal voids of the microspheres of the present disclosure is about 0.016 to 0.066 mL/g.

In some embodiments, 50% of the cumulative pore volume of the internal voids of the microspheres of the present disclosure is about 0.013 to 0.11 mL/g, 0.014 to 0.10 mL/g, 0.015 to 0.09 mL/g, or 0.016 to 0.08 mL/g.

In some embodiments, 10% of the cumulative pore volume of the internal voids of the microspheres of the present disclosure is about 0.009 to 0.016 mL/g.

In some embodiments, 10% of the cumulative pore volume of the internal voids of the microspheres of the present disclosure is about 0.011 to 0.014 mL/g.

In some embodiments, 10% of the cumulative pore volume of the internal voids of the microspheres of the present disclosure is about 0.011 to 0.022 mL/g, 0.011 to 0.021 mL/g, 0.011 to 0.020 mL/g, 0.011 to 0.019 mL/g, 0.011 to 0.018 mL/g, 0.011 to 0.017 mL/g, or 0.011 to 0.016 mL/g.

In some embodiments, semaglutide and sucrose are dissolved in water to obtain a first aqueous solution.

In some embodiments, a pharmaceutically acceptable polymer is dissolved in an organic solvent to give an oil solution.

In some embodiments, exemplary examples of organic solvents that can be used in the present disclosure include, but are not limited to, dichloromethane, ethyl acetate and acetone.

In some embodiments, the first aqueous solution and the oil solution are emulsified by homogenous or ultrasonic means to obtain a water-in-oil (W/O) emulsion.

In some embodiments, the emulsifier and the osmotic pressure modifier are dissolved in water to obtain a second aqueous solution.

In some embodiments, exemplary examples of emulsifiers that can be used in the present disclosure include, but are not limited to, anionic surfactants and nonionic surfactants.

In some embodiments, exemplary examples of anionic surfactants that can be used in the present disclosure include, but are not limited to, sodium oleate, sodium stearate and sodium lauryl sulfate.

In some embodiments, exemplary examples of nonionic surfactants that can be used in the present disclosure include, but are not limited to, polyoxyethylene sorbitol fatty acid esters, polyoxyethylene castor oil derivatives, polyvinylpyrrolidone, polyvinyl alcohol, carboxymethylcellulose, lecithin and gelatin.

In some embodiments, exemplary examples of polyoxyethylene sorbitol fatty acid esters that can be used in the present disclosure include, but are not limited to, Tween 80 and Tween 60.

In some embodiments, exemplary examples of osmotic pressure modifiers that can be used in the present disclosure include, but are not limited to, sodium chloride, potassium chloride, mannitol, sorbitol, glucose and proline.

In some embodiments, polyvinyl alcohol and sodium chloride are dissolved in water to obtain a second aqueous solution.

In some embodiments, the water-in-oil (W/O) emulsion is mixed with a second aqueous solution using cantilever mixer, homogenizer, or static mixer to obtain a water-in-oil-in-water (W/O/W) emulsion.

In some embodiments, a gas is used to purge the W/O/W emulsion, thereby removing the solvent in the oil phase of the W/O/W emulsion, which results in hardened microspheres.

In some embodiments, exemplary examples of gases that can be used in the present disclosure include, but are not limited to, nitrogen, helium, compressed air, argon and neon.

In some embodiments, the W/O/W emulsion is purged with gas while stirring to remove the solvent in the oil phase of the W/O/W emulsion, which results in hardened microspheres.

In some embodiments, exemplary examples of agitation modes that can be used in the present disclosure include, but are not limited to, mechanical agitation and magnetic levitation agitation.

In some embodiments, exemplary examples of mechanical agitation that can be used in the present disclosure include, but are not limited to, cantilever paddles.

In some embodiments, the droplet in water-in-oil (W/O) emulsion has a particle size of about 100 to 200 nm.

In some embodiments, the droplet in water-in-oil (W/O) emulsion has a particle size of about 110 to 200 nm.

In some embodiments, the droplet in water-in-oil (W/O) emulsion has a particle size of about 120 to 200 nm.

In some embodiments, the droplet in water-in-oil (W/O) emulsion has a particle size of about 130 to 200 nm.

In some embodiments, the droplet in water-in-oil (W/O) emulsion has a particle size of about 140 to 200 nm.

In some embodiments, the droplet in water-in-oil (W/O) emulsion has a particle size of about 150 to 200 nm.

In some embodiments, the droplet in water-in-oil (W/O) emulsion has a particle size of about 160 to 200 nm.

In some embodiments, the droplet in water-in-oil (W/O) emulsion has a particle size of about 170 to 200 nm.

In some embodiments, the droplet in water-in-oil (W/O) emulsion has a particle size of about 180 to 200 nm.

In some embodiments, the droplet in water-in-oil (W/O) emulsion has a particle size of about 190 to 200 nm.

In some embodiments, the hardened microspheres are rinsed.

In some embodiments, exemplary examples of rinsing solutions that can be used in the present disclosure include, but are not limited to, water for injection and purified water.

In some embodiments, the rinsed microspheres are lyophilized to yield a microsphere lyophilized powder.

In yet another aspect, the present disclosure relates to microspheres prepared by a process comprising: providing a first aqueous solution comprising semaglutide; providing an oil solution containing a pharmaceutically acceptable polymer; emulsifying the first aqueous solution and the oil solution to obtain a water-in-oil (W/O) emulsion; mixing the water-in-oil (W/O) emulsion with a second aqueous solution to obtain a W/O/W emulsion; and removing solvent in oil phase of the W/O/W emulsion to obtain the microspheres, wherein drug loading of the microspheres is about 11.0% to 12.5% and 90% of cumulative pore volume of internal voids of the microspheres is about 0.013 to 0.15 mL/g.

In a further aspect, the present disclosure relates to microspheres prepared by a process comprising: providing a first aqueous solution comprising semaglutide; providing an oil solution containing a pharmaceutically acceptable polymer; emulsifying the first aqueous solution and the oil solution to obtain a water-in-oil (W/O) emulsion; mixing the water-in-oil (W/O) emulsion with a second aqueous solution to obtain a water-in-oil-in-water (W/O/W) emulsion; and removing solvent in oil phase of the W/O/W emulsion to obtain the microspheres, wherein drug loading of the microspheres is about 11.0% to 20.0% and 90% of cumulative pore volume of internal voids of the microspheres is about 0.013 to 0.20mL/g.

In some embodiments, the drug loading of the microspheres of the present disclosure is about 11.1%.

In some embodiments, the drug loading of the microspheres of the present disclosure is about 11.2%.

In some embodiments, the drug loading of the microspheres of the present disclosure is about 11.3%.

In some embodiments, the drug loading of the microspheres of the present disclosure is about 11.4%.

In some embodiments, the drug loading of the microspheres of the present disclosure is about 11.5%.

In some embodiments, the drug loading of the microspheres of the present disclosure is about 11.6%.

In some embodiments, the drug loading of the microspheres of the present disclosure is about 11.7%.

In some embodiments, the drug loading of the microspheres of the present disclosure is about 11.8%.

In some embodiments, the drug loading of the microspheres of the present disclosure is about 11.9%.

In some embodiments, the drug loading of the microspheres of the present disclosure is about 12.0%.

In some embodiments, the drug loading of the microspheres of the present disclosure is about 12.1%.

In some embodiments, the drug loading of the microspheres of the present disclosure is about 12.2%.

In some embodiments, the drug loading of the microspheres of the present disclosure is about 12.3%.

In some embodiments, the drug loading of the microspheres of the present disclosure is about 12.4%.

In some embodiments, the microspheres of the present disclosure have a drug loading of about 12.5%, 12.6%, 12.7%, 12.8%, 12.9%, 13.0%, 13.1%, 13.2%, 13.3%, 13.4%, 13.5%, 13.6%, 13.7%, 13.8%, 13.9%, 14.0%, 14.1%, 14.2%, 14.3%, 14.4%, 14.5%, 14.6%, 14.7%, 14.8%, 14.9%, 15.0%, 15.1%, 15.2%, 15.3%, 15.4%, 15.5%, 15.6%, 15.7%, 15.8%, 15.9%, 16.0%, 16.1%, 16.2%, 16.3%, 16.4%, 16.5%, 16.6%, 16.7%, 16.8%, 16.9%, 17.0%, 17.1%, 17.2%, 17.3%, 17.4%, 17.5%, 17.6%, 17.7%, 17.8%, 17.9%, 18.0%, 18.1%, 18.2%, 18.3%, 18.4%, 18.5%, 18.6%, 18.7%, 18.8%, 18.9%, 19.0%, 19.1%, 19.2%, 19.3%, 19.4%, 19.5%, 19.6%, 19.7%, 19.8%, 19.9%, or 20.0%.

In some embodiments, the drug loading of the microspheres of the present disclosure is about 11.0% to 19.0%, or 11.0% to 18.0%.

In some embodiments, 90% of the cumulative pore volume of the internal voids of the microspheres of the present disclosure is about 0.017 to 0.12 mL/g.

In some embodiments, 90% of the cumulative pore volume of the internal voids of the microspheres of the present disclosure is about 0.014 to 0.19mL/g, 0.015 to 0.18mL/g, 0.016 to 0.17mL/g, 0.017 to 0.18mL/g, 0.017 to 0.17mL/g, 0.017 to 0.16mL/g, or 0.017 to 0.15 mL/g.

In some embodiments, 50% of the cumulative pore volume of the internal voids of the microspheres of the present disclosure is about 0.013 to 0.079 mL/g.

In some embodiments, 50% of the cumulative pore volume of the internal voids of the microspheres of the present disclosure is about 0.016 to 0.066 mL/g.

In some embodiments, 50% of the cumulative pore volume of the internal voids of the microspheres of the present disclosure is about 0.013 to 0.11 mL/g, 0.014 to 0.10 mL/g, 0.015 to 0.09 mL/g, or 0.016 to 0.08 mL/g.

In some embodiments, 10% of the cumulative pore volume of the internal voids of the microspheres of the present disclosure is about 0.009 to 0.016 mL/g.

In some embodiments, 10% of the cumulative pore volume of the internal voids of the microspheres of the present disclosure is about 0.011 to 0.014 mL/g.

In some embodiments, 10% of the cumulative pore volume of the internal voids of the microspheres of the present disclosure is about 0.011 to 0.022 mL/g, 0.011 to 0.021 mL/g, 0.011 to 0.020 mL/g, 0.011 to 0.019 mL/g, 0.011 to 0.018 mL/g, 0.011 to 0.017 mL/g, or 0.011 to 0.016 mL/g.

In some embodiments, the droplet in water-in-oil (W/O) emulsion has a particle size of about 100 to 200 nm.

In some embodiments, the droplet in water-in-oil (W/O) emulsion has a particle size of about 110 to 200 nm.

In some embodiments, the droplet in water-in-oil (W/O) emulsion has a particle size of about 120 to 200 nm.

In some embodiments, the droplet in water-in-oil (W/O) emulsion has a particle size of about 130 to 200 nm.

In some embodiments, the droplet in water-in-oil (W/O) emulsion has a particle size of about 140 to 200 nm.

In some embodiments, the droplet in water-in-oil (W/O) emulsion has a particle size of about 150 to 200 nm.

In some embodiments, the droplet in water-in-oil (W/O) emulsion has a particle size of about 160 to 200 nm.

In some embodiments, the droplet in water-in-oil (W/O) emulsion has a particle size of about 170 to 200 nm.

In some embodiments, the droplet in water-in-oil (W/O) emulsion has a particle size of about 180 to 200 nm.

In some embodiments, the droplet in water-in-oil (W/O) emulsion has a particle size of about 190 to 200 nm.

In some embodiments, the microspheres of the present disclosure have a higher encapsulation efficiency. Furthermore, the microsphere encapsulation efficiency of the present disclosure is higher than 80%, even higher than 85%.

In some embodiments, the microspheres of the present disclosure have improved bioavailability.

In some embodiments, the microspheres of the present disclosure have an improved AUC_{inf}.

In some embodiments, the microspheres of the present disclosure have an ideally acceptable low burst amount, which in turn is beneficial in maintaining stable plasma concentrations *in vivo* after administration.

In some embodiments, the amount of microspheres of the present disclosure when administered to a subject in need thereof may be less, the volume of administration may be lower, and still have excellent bioavailability. Furthermore, lower microsphere administration amounts and lower administration volumes may reduce local irritation responses and reduce patient pain sensation.

In some embodiments, the release period of semaglutide from the microspheres of the present disclosure is about 2 weeks or more.

In some embodiments, semaglutide is sustainedly released from microspheres of the present disclosure.

In some aspect, the present disclosure relates to a method for treating diabetes, comprising administering to a subject in need thereof a therapeutically effective amount of the microspheres described in the present disclosure, the pharmaceutical composition described in the present disclosure, or the microspheres prepared by the process described in the present disclosure.

In some embodiments, the microspheres described in the present disclosure, the pharmaceutical compositions described in the present disclosure, or the microspheres prepared by the process described in the present disclosure are administered to a subject at a frequency of 2 weeks or more.

In some embodiments, the diabetes is type 2 diabetes.

In some embodiments, exemplary examples of individuals that can be used in the present disclosure include, but are not limited to, mammals.

In some embodiments, exemplary examples of mammals that can be used in the present disclosure include, but are not limited to, humans.

In a further aspect, the present disclosure relates to a method for reducing body weight, comprising administering to a subject in need thereof an effective amount of the microspheres described in the present disclosure, the pharmaceutical composition described in the present disclosure, or the microspheres prepared by the process described in the present disclosure.

In some embodiments, the microspheres described in the present disclosure, the pharmaceutical compositions described in the present disclosure, or the microspheres prepared by the process described in the present disclosure are administered to a subject at a frequency of 2 weeks or more.

In some embodiments, exemplary examples of individuals that can be used in the present disclosure include, but are not limited to, mammals.

In some embodiments, exemplary examples of mammals that can be used in the present disclosure include, but are not limited to, humans.

Hereinafter, the present disclosure will be explained in detail by the following examples in order to better understand various aspects of the present application and advantages thereof. However, it is to be understood that the following examples are non-limiting and are intended to illustrate only certain embodiments of the present disclosure.

### EXAMPLES

The reagents and devices used in the embodiments of the present disclosure are both conventional and commercially available. For example:

| Materials | Manufacturers |
|---|---|
| Semaglutide | Shenzhen JYMed Technology Co., Ltd. |

| | |
|---|---|
| PLGA | Evonik Industries AG |
| Sucrose | Merck |
| Dichloromethane | Tianjin Kemiou Chemical Reagent Co., Ltd. |
| PVA | Merck |
| Sodium chloride | SinoPharm Chemical Reagent Co., Ltd. |

| Main Devices | Manufacturers |
|---|---|
| Electronic balance | Mettler Toledo Technology (China) Co., Ltd. |
| Electronic balance | Mettler Toledo Technology (China) Co., Ltd. |
| Economic precise high-temperature constant-temperature liquid bath | Hangzhou Xutemp Temptech Co., Ltd. |
| High performance liquid chromatograph | Walters Science & Technology (Shanghai) Co., Ltd. |
| Cell ultrasonic breaker | Ningbo Xinzhi Biotechnology Co., Ltd. |
| High-speed shearing machine | Ika (Guangzhou) Instrument Equipment Co., Ltd. |
| Overhead cantilever mixer | Ika (Guangzhou) Instrument Equipment Co., Ltd. |
| Nanometer particle size and Zeta potential analyzer | Malvern Instruments Limited, UK |

### Example 1

### Preparation of Semaglutide Microspheres

| Composition | Input amount/g | Percentage/% |
|---|---|---|
| Semaglutide | 1.0116 | 13.21 |
| PLGA | 6.4966 | 84.82 |
| Sucrose | 0.1511 | 1.97 |
| Amount of water for injection in internal aqueous phase * | 4.1588 | - |
| Amount of dichloromethane in oil phase * | 101.9 | - |
| Amount of water for injection in external aqueous phase * | 10139.0 | - |
| PVA * | 50.95 | - |
| Sodium chloride * | 101.90 | - |
| Total | 7.6593 | 100.00 |

| | | |
|---|---|---|
| Note: * Removed during preparation | | |

Preparation Procedure:
1. An amount of semaglutide and sucrose were dissolved in water to give an internal aqueous phase (i.e., a first aqueous solution).
2. An amount of PLGA was dissolved in dichloromethane to give an oil phase.
3. An amount of PVA and sodium chloride were dissolved in water to give an external aqueous phase (i.e., a second aqueous solution).
4. The internal aqueous phase and the oil phase were emulsified by ultrasonic manner to obtain a droplet in water-in-oil emulsion having a particle size of 160.6 nm.
5. The water-in-oil emulsion obtained in the above-mentioned step 4 was mixed with a certain proportion of external aqueous phase in a homogeneous manner to obtain a water-in-oil-in-water system.
6. In the presence of mechanical agitation, the surface of the water-in-oil-in-water system obtained in the above-mentioned step 5 was purged with nitrogen, the dichloromethane dissolved in the external aqueous phase was volatilized and the microspheres were hardened.
7. The hardened microspheres were collected and washed with purified water.
8. The wet microspheres obtained in the above-mentioned step 7 were collected and lyophilized.
9. The freeze-dried powder of the microspheres obtained in the step 8 was collected, sieved and dispersed.

### Example 2

### Preparation of Semaglutide Microspheres

| Composition | Input amount/g | Percentage/% |
|---|---|---|
| Semaglutide | 3.1208 | 13.21 |
| PLGA | 20.0337 | 84.80 |
| Sucrose | 0.4708 | 1.99 |
| Amount of water for injection in internal aqueous phase * | 12.558 | - |
| Amount of dichloromethane in oil phase * | 313.30 | - |
| Amount of water for injection in external aqueous phase * | 12469.3 | - |
| PVA * | 62.66 | - |
| Sodium chloride * | 125.3 | - |
| Total | 23.625 | 100.00 |

| | | |
|---|---|---|
| Note: * Removed during preparation | | |

Preparation Procedure:
1. An amount of semaglutide drug substance and sucrose were dissolved in water to give an internal aqueous phase (i.e., a first aqueous solution).
2. An amount of PLGA was dissolved in dichloromethane to give an oil phase.
3. An amount of PVA and sodium chloride were dissolved in water to give an external aqueous phase (i.e., a second aqueous solution).
4. The internal aqueous phase and the oil phase were emulsified by ultrasonic manner to obtain a droplet in water-in-oil emulsion having a particle size of 180.8 nm.
5. The water-in-oil emulsion obtained in the above-mentioned step 4 was mixed with a certain proportion of external aqueous phase in a homogeneous manner to obtain a water-in-oil-in-water system.
6. In the presence of mechanical agitation, the surface of the water-in-oil-in-water system obtained in the above-mentioned step 5 was purged with nitrogen, the dichloromethane dissolved in the external aqueous phase was volatilized and the microspheres were hardened.
7. The hardened microspheres were collected and washed with purified water.
8. The wet microspheres obtained in the above-mentioned step 7 were collected and lyophilized.
9. The freeze-dried powder of the microspheres obtained in the step 8 was collected, sieved and dispersed.

### Example 3

### Preparation of Semaglutide Microspheres

| Composition | Input amountg | Percentage/% |
|---|---|---|
| Semaglutide | 1.3972 | 13.18 |
| PLGA | 8.9905 | 84.83 |
| Sucrose | 0.2105 | 1.99 |
| Amount of water for injection in internal aqueous phase * | 5.6515 | - |
| Amount of dichloromethane in oil phase * | 140.9 | - |
| Amount of water for injection in external aqueous phase * | 14019.55 | - |
| PVA * | 70.45 | - |
| Sodium chloride * | 140.9 | - |
| Total | 10.5982 | 100.00 |

| | | |
|---|---|---|
| Note: * Removed during preparation | | |

Preparation Procedure:
1. An amount of semaglutide drug substance and sucrose were dissolved in water to give an internal aqueous phase (i.e., a first aqueous solution).
2. An amount of PLGA was dissolved in dichloromethane to give an oil phase.
3. An amount of PVA and sodium chloride were dissolved in water to give an external aqueous phase (i.e., a second aqueous solution).
4. The internal aqueous phase and the oil phase were emulsified by ultrasonic manner to obtain a droplet in water-in-oil emulsion having a particle size of 269.3 nm.
5. The water-in-oil emulsion obtained in the above-mentioned step 4 was mixed with a certain proportion of external aqueous phase in a homogeneous manner to obtain a water-in-oil-in-water system.
6. In the presence of mechanical agitation, the surface of the water-in-oil-in-water system obtained in the above-mentioned step 5 was purged with nitrogen, the dichloromethane dissolved in the external aqueous phase was volatilized, and the microspheres were hardened.
7. The hardened microspheres were collected and washed with purified water.
8. The wet microspheres obtained in the above-mentioned step 7 were collected and lyophilized.
9. The freeze-dried powder of the microspheres obtained in the step 8 was collected, sieved and dispersed.

### Example 4

### Preparation of Semaglutide Microspheres

| Composition | Input amount/g | Percentage/% |
|---|---|---|
| Semaglutide | 0.6044 | 5.58 |
| PLGA | 10.0124 | 92.41 |
| Sucrose | 0.2175 | 2.01 |
| Amount of water for injection in internal aqueous phase * | 6.2833 | - |
| Amount of dichloromethane in oil phase * | 157.22 | - |
| Amount of water for injection in external aqueous phase * | 15643.0 | - |
| PVA * | 78.61 | - |
| Sodium chloride * | 157.22 | - |
| Total | 10.8343 | 100.00 |

| | | |
|---|---|---|
| Note: * Removed during preparation | | |

Preparation Procedure:
1. An amount of semaglutide drug substance and sucrose were dissolved in water to give an internal aqueous phase (i.e., a first aqueous solution).
2. An amount of PLGA was dissolved in dichloromethane to give an oil phase.
3. An amount of PVA and sodium chloride were dissolved in water to give an external aqueous phase (i.e., a second aqueous solution).
4. The internal aqueous phase and the oil phase were emulsified by ultrasonic manner to obtain a droplet in water-in-oil emulsion having a particle size of 151.5 nm.
5. The water-in-oil emulsion obtained in the above-mentioned step 4 was mixed with a certain proportion of external aqueous phase in a homogeneous manner to obtain a water-in-oil-in-water system.
6. In the presence of mechanical agitation, the surface of the water-in-oil-in-water system obtained in the above-mentioned step 5 was purged with nitrogen, the dichloromethane dissolved in the external aqueous phase was volatilized, and the microspheres were hardened.
7. The hardened microspheres were collected and washed with purified water.
8. The wet microspheres obtained in the above-mentioned step 7 were collected and lyophilized.
9. The freeze-dried powder of the microspheres obtained in the step 8 was collected, sieved and dispersed.

### Example 5

### Preparation of Semaglutide Microspheres

| Composition | Input amount/g | Percentage/% |
|---|---|---|
| Semaglutide | 0.7537 | 19.64 |
| PLGA | 3.0087 | 78.40 |
| Sucrose | 0.07537 | 1.96 |
| Amount of water for injection in internal aqueous phase * | 4.7176 | - |
| Amount of dichloromethane in oil phase * | 47.10 | - |
| Amount of water for injection in external aqueous phase * | 1874.6 | - |
| PVA * | 9.42 | - |
| Sodium chloride * | 18.84 | - |
| Total | 3.8378 | 100.00 |

| | | |
|---|---|---|
| Note: * Removed during preparation | | |

Preparation Procedure:
1. An amount of semaglutide drug substance and sucrose were dissolved in water to give an internal aqueous phase (i.e., a first aqueous solution).
2. An amount of PLGA was dissolved in dichloromethane to give an oil phase.
3. An amount of PVA and sodium chloride were dissolved in water to give an external aqueous phase (i.e., a second aqueous solution).
4. The internal aqueous phase and the oil phase were emulsified by ultrasonic manner to obtain a droplet in water-in-oil emulsion having a particle size of 175 nm.
5. The water-in-oil emulsion obtained in the above-mentioned step 4 was mixed with a certain proportion of external aqueous phase in a homogeneous manner to obtain a water-in-oil-in-water system.
6. In the presence of mechanical agitation, the surface of the water-in-oil-in-water system obtained in the above-mentioned step 5 was purged with air, dichloromethane dissolved in the external aqueous phase was volatilized, and the microspheres were hardened.
7. The hardened microspheres were collected and washed with purified water.
8. The wet micorspheres obtained in the above-mentioned step 7 were collected and lyophilized.
9. The freeze-dried powder of the microspheres obtained in the step 8 was collected, sieved and dispersed.

### Example 6

### Preparation of Semaglutide Microspheres

| Composition | Input amount/g | Percentage/% |
|---|---|---|
| Semaglutide | 1.143 | 26.99 |
| PLGA | 3.0096 | 71.06 |
| Sucrose | 0.0829 | 1.96 |
| Amount of water for injection in internal aqueous phase * | 4.7294 | - |
| Amount of dichloromethane in oil phase * | 47.00 | - |
| Amount of water for injection in external aqueous phase * | 1871.0 | - |
| PVA * | 9.408 | - |
| Sodium chloride * | 18.8009 | - |
| Total | 4.2355 | 100 |

| | | |
|---|---|---|
| Note: * Removed during preparation | | |

Preparation Procedure:
1. An amount of semaglutide drug substance and sucrose were dissolved in water to give an internal aqueous phase (i.e., a first aqueous solution).
2. An amount of PLGA was dissolved in dichloromethane to give an oil phase.
3. An amount of PVA and sodium chloride were dissolved in water to give an external aqueous phase (i.e., a second aqueous solution).
4. The internal aqueous phase and the oil phase were emulsified by ultrasonic manner to obtain a droplet in water-in-oil emulsion having a particle size of 183.4 nm.
5. The water-in-oil emulsion obtained in the above-mentioned step 4 was mixed with a certain proportion of external aqueous phase in a homogeneous manner to obtain a water-in-oil-in-water system.
6. In the presence of mechanical agitation, the surface of the water-in-oil-in-water system obtained in the above-mentioned step 5 was purged with air, dichloromethane dissolved in the external aqueous phase was volatilized, and the microspheres were hardened.
7. The hardened microspheres were collected and washed with purified water.
8. The wet microspheres obtained in the above-mentioned step 7 were collected and lyophilized.
9. The freeze-dried powder of the microspheres obtained in the step 8 was collected, sieved and dispersed.

### Example 7

### Determination of Release of Semaglutide Microspheres

### Sample to be tested

Semaglutide microspheres prepared in Examples 1 to 6.

Three parallel samples were set for each sample.

The release rate was measured by XT5018P-GP18 Economic precise high-temperature constant-temperature liquid bath and Waters high performance liquid chromatograph.

### Preparation of release medium

Tris (base) 34.06±0.01 g, Tris (HCl) 2.97±0.01 g, poloxamer (188) 5±0.1 g, sodium azide 1.00±0.01 g were placed in a suitable container, stirred with 1 L of purified water, adjusted to pH 9.40±0.03 with 0.1 mol/L of sodium hydroxide, and filtered through a steam-sterilized capsule filter (BP milpak 20, PVDF, 0.22 µm) to obtain the release medium.

About 50 mg of semaglutide microspheres were taken, accurately weighed, and placed in a 50 mL blue cap bottle. 20 mL of the release medium was added and the microspheres were suspended by shaking and then placed in a constant temperature water bath (37°C±0.3°C). The samples were removed at 1 d, 3 d, 7 d, 10 d, 14 d, 17 d, 21 d, 24 d, 28 d, 31 d, 35 d, and 42 d after standing. 1 mL of the supernatant was taken with a microsampler and centrifuged in a 2 mL centrifuge tube (5000 rpm for 10 min). An equal amount of the preheated fresh medium was added after sampling. The supernatant was taken in a suitable amount after centrifugation. Three parallel samples were prepared.

### Chromatographic conditions

Column: Waters Xselect^{®} CSH C18 3.5 µm, 4.6×150 mm
Column temperature: 45°C
Mobile phase: 0.05 mol/L ammonium dihydrogen phosphate (pH 9.50) - acetonitrile (2: 1)
Flow rate: 1.0 mL/min
Detection wavelength: 215 nm
Sample Tray Temperature: 10°C
Injection volume: 10 µL
Elution mode: Isocratic elution
Run time: 10 min

The cumulative drug release over time of the microspheres of Examples 1 to 6 of the present disclosure were shown in Tables 1 to 6.

**Table 1.**

| Example 1 | Sample 1 | Sample 2 | Sample 3 | Mean Value | RSD |
|---|---|---|---|---|---|
| 0.04 d | 0.39% | 0.56% | 0.47% | 0.47% | 17.94% |
| 0.17 d | 1.01% | 0.93% | 0.64% | 0.86% | 22.14% |
| 1 d | 6.78% | 6.81% | 6.79% | 6.79% | 0.23% |
| 3 d | 14.24% | 13.71% | 14.17% | 14.04% | 2.04% |
| 7 d | 24.40% | 24.62% | 24.19% | 24.40% | 0.88% |
| 10 d | 31.13% | 30.52% | 30.85% | 30.83% | 0.99% |
| 14 d | 32.51% | 32.92% | 31.73% | 32.39% | 1.86% |
| 17 d | 40.86% | 40.97% | 41.16% | 41.00% | 0.38% |
| 21 d | 45.87% | 46.42% | 46.26% | 46.18% | 0.61% |
| 24 d | 50.57% | 50.97% | 50.93% | 50.82% | 0.43% |
| 27 d | 62.98% | 67.41% | 67.77% | 66.05% | 4.04% |
| 31 d | 79.52% | 85.73% | 85.29% | 83.51% | 4.15% |
| 35 d | 93.28% | 100.82% | 98.46% | 97.52% | 3.95% |

| | | | | | |
|---|---|---|---|---|---|
| Note: D is day | | | | | |

**Table 2.**

| Example 2 | Sample 1 | Sample 2 | Sample 3 | Mean Value | RSD |
|---|---|---|---|---|---|
| 0.04 d | 0.58% | 0.37% | 0.38% | 0.38% | 31.05% |
| 0.17 d | 0.38% | 0.32% | 0.45% | 0.38% | 17.07% |
| 1 d | 4.82% | 5.11% | 4.66% | 4.86% | 4.71% |
| 3 d | 12.81% | 12.37% | 12.30% | 12.49% | 2.19% |
| 7 d | 26.79% | 29.44% | 30.52% | 28.92% | 6.63% |
| 10 d | 33.12% | 33.01% | 32.94% | 33.02% | 0.27% |
| 14 d | 39.23% | 39.22% | 39.52% | 39.32% | 0.43% |
| 17 d | 44.26% | 44.06% | 44.52% | 44.28% | 0.52% |
| 21 d | 50.63% | 51.01% | 51.74% | 51.13% | 1.11% |
| 24 d | 56.40% | 57.35% | 58.47% | 57.41% | 1.81% |
| 27 d | 77.98% | 78.69% | 79.87% | 78.85% | 1.21% |
| 31 d | 82.54% | 89.70% | 90.96% | 87.73% | 5.18% |
| 35 d | 83.95% | 89.03% | 90.78% | 87.92% | 4.04% |

| | | | | | |
|---|---|---|---|---|---|
| Note: D is day | | | | | |

**Table 3.**

| Example 3 | Sample 1 | Sample 2 | Sample 3 | Mean Value | RSD |
|---|---|---|---|---|---|
| 0.04 d | 13.16% | 13.27% | 13.09% | 13.17% | 0.70% |
| 0.17 d | 14.54% | 14.58% | 14.71% | 14.61% | 0.61% |
| 1 d | 20.64% | 21.05% | 21.29% | 21.00% | 1.57% |
| 3 d | 23.25% | 23.73% | 24.68% | 23.89% | 3.05% |
| 7 d | 28.23% | 28.79% | 29.36% | 28.79% | 1.96% |
| 10 d | 33.42% | 34.48% | 33.76% | 33.88% | 1.59% |
| 14 d | 34.91% | 35.52% | 35.65% | 35.36% | 1.12% |
| 17 d | 36.31% | 36.35% | 37.34% | 36.67% | 1.60% |
| 21 d | 37.76% | 38.23% | 38.42% | 38.13% | 0.89% |
| 24 d | 38.94% | 40.06% | 40.08% | 39.70% | 1.64% |
| 27 d | 46.85% | 52.96% | 46.79% | 48.87% | 7.26% |
| 31 d | 71.28% | 82.11% | 76.78% | 76.72% | 7.06% |
| 35 d | 93.91% | 101.95% | 101.00% | 98.95% | 4.44% |

| | | | | | |
|---|---|---|---|---|---|
| Note: D is day | | | | | |

**Table 4.**

| Example 4 | Sample 1 | Sample 2 | Sample 3 | Mean Value | RSD |
|---|---|---|---|---|---|
| 0.04 d | 0.44% | 0.56% | 0.51% | 0.50% | 11.47% |
| 0.17 d | 0.36% | 0.56% | 0.42% | 0.49% | 13.72% |
| 1 d | 4.11% | 4.15% | 3.81% | 4.02% | 4.58% |
| 3 d | 9.11% | 9.96% | 9.16% | 9.41% | 5.04% |
| 7 d | 16.59% | 17.52% | 17.25% | 17.12% | 2.82% |
| 10 d | 19.40% | 20.78% | 22.13% | 20.77% | 6.57% |
| 14 d | 22.28% | 23.07% | 22.42% | 22.59% | 1.87% |
| 17 d | 24.17% | 26.52% | 24.79% | 25.16% | 4.83% |
| 21 d | 29.66% | 32.98% | 31.95% | 31.53% | 5.39% |
| 24 d | 33.87% | 38.87% | 33.96% | 35.57% | 8.05% |
| 27 d | 50.52% | 54.28% | 50.01% | 51.60% | 4.52% |
| 31 d | 69.85% | 70.97% | 69.14% | 69.98% | 1.32% |
| 35 d | 80.96% | 87.96% | 77.01% | 81.98% | 6.76% |
| 42 d | 97.99% | 105.33% | 85.36% | 96.22% | 10.50% |

| | | | | | |
|---|---|---|---|---|---|
| Note: D is day | | | | | |

**Table 5.**

| Example 5 | Sample 1 | Sample 2 | Sample 3 | Mean Value | RSD |
|---|---|---|---|---|---|
| 0.04 d | 0.44% | 0.56% | 0.51% | 0.50% | 11.47% |
| 0.17 d | 0.36% | 0.56% | 0.42% | 0.49% | 13.72% |
| 1 d | 4.11% | 4.15% | 3.81% | 4.02% | 4.58% |
| 3 d | 9.11% | 9.96% | 9.16% | 9.41% | 5.04% |
| 7 d | 16.59% | 17.52% | 17.25% | 17.12% | 2.82% |
| 10 d | 19.40% | 20.78% | 22.13% | 20.77% | 6.57% |
| 14 d | 22.28% | 23.07% | 22.42% | 22.59% | 1.87% |
| 17 d | 24.17% | 26.52% | 24.79% | 25.16% | 4.83% |
| 21 d | 29.66% | 32.98% | 31.95% | 31.53% | 5.39% |
| 24 d | 33.87% | 38.87% | 33.96% | 35.57% | 8.05% |
| 27 d | 50.52% | 54.28% | 50.01% | 51.60% | 4.52% |
| 31 d | 69.85% | 70.97% | 69.14% | 69.98% | 1.32% |
| 35 d | 80.96% | 87.96% | 77.01% | 81.98% | 6.76% |
| 42 d | 97.99% | 105.33% | 85.36% | 96.22% | 10.50% |

| | | | | | |
|---|---|---|---|---|---|
| Note: D is day | | | | | |

**Table 6.**

| Example 7 | Sample 1 | Sample 2 | Sample 3 | Mean Value | RSD |
|---|---|---|---|---|---|
| 0.04 d | 6.52% | 6.19% | 6.46% | 6.39% | 2.77% |
| 0.17 d | 8.09% | 7.46% | 7.82% | 7.79% | 4.06% |
| 1 d | 12.32% | 11.67% | 11.74% | 11.91% | 2.98% |
| 3 d | 20.17% | 19.44% | 19.52% | 19.71% | 2.02% |
| 7 d | 29.63% | 27.84% | 27.96% | 28.48% | 3.52% |
| 10 d | 35.25% | 33.32% | 34.07% | 34.21% | 2.85% |
| 14 d | 44.05% | 41.85% | 42.46% | 42.79% | 2.65% |
| 17 d | 50.81% | 48.64% | 48.25% | 49.23% | 2.80% |
| 21 d | 62.55% | 58.75% | 59.67% | 60.32% | 3.28% |
| 24 d | 82.48% | 76.62% | 75.43% | 78.18% | 4.82% |
| 28 d | 91.14% | 87.76% | 86.81% | 88.57% | 2.57% |
| 31 d | 92.11% | 88.92% | 88.12% | 89.72% | 2.35% |
| 35 d | 92.45% | 89.37% | 88.38% | 90.07% | 2.36% |

| | | | | | |
|---|---|---|---|---|---|
| Note: D is day | | | | | |

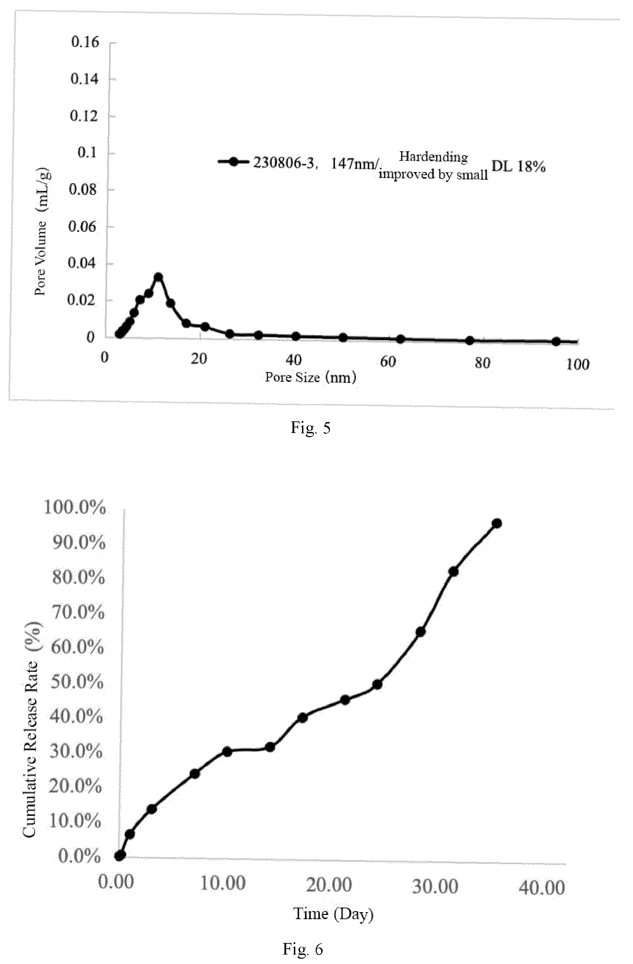

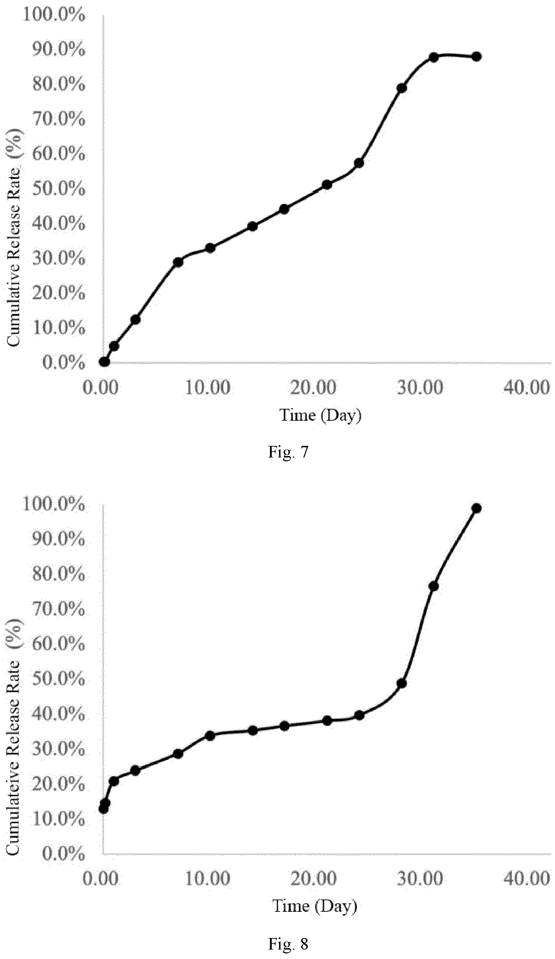

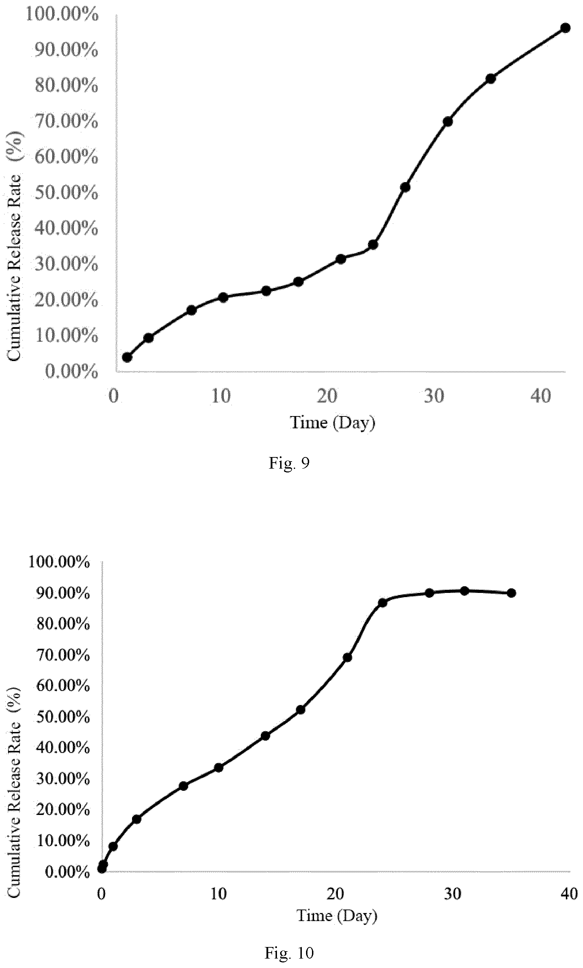

Figs. 6 to 11 showed the cumulative release profiles of the microspheres of Examples 1 to 6 of the present disclosure.

### Example 8

### Determination of Drug Loading of Semaglutide Microspheres

### Sample to be tested

Semaglutide microspheres prepared in Examples 1 to 6.

Three parallel samples were set for each sample.

### Detection method

1. PLGA in the microspheres was dissolved with acetonitrile to release semaglutide. Semaglutide was then dissolved in phosphate buffer and most of the PLGA was precipitated and finally removed by centrifugation.
2. The content of semaglutide was determined by HPLC.

| Examples | Drug Loading |
|---|---|
| Example 1 | 11.44% |
| Example 2 | 11.78% |
| Example 3 | 11.51% |
| Example 4 | 4.94% |
| Example 5 | 17.2% |
| Example 6 | 21.78% |

### Example 9

### Determination of Pore Size Distribution of Microspheres

About 200 mg of semaglutide microspheres were weighed in a dilatometer, sealed, and weighed to obtain a dilatometer assembly weight. The machine was subjected to a low pressure test and a high pressure test. The pressure was increased from 0.50 psia to 60000 psia.

Samples to be tested: Semaglutide microspheres prepared in Examples 1 to 5.

The pore volume and pore size distribution of the samples were calculated from the mercury intake curves obtained using the Washburn equation.

| Examples | 10% of cumulative pore volume of internal voids of microspheres (mL/g) | 50% of cumulative pore volume of internal voids of microspheres (mL/g) | 90% of cumulative pore volume of internal voids of microspheres (mL/g) |
|---|---|---|---|
| 1 | 0.0132 | 0.0658 | 0.1185 |
| 2 | 0.0116 | 0.0166 | 0.0173 |
| 3 | 0.0295 | 0.1473 | 0.2652 |
| 4 | 0.0109 | 0.0545 | 0.0981 |
| 5 | 0.0160 | 0.0800 | 0.1439 |

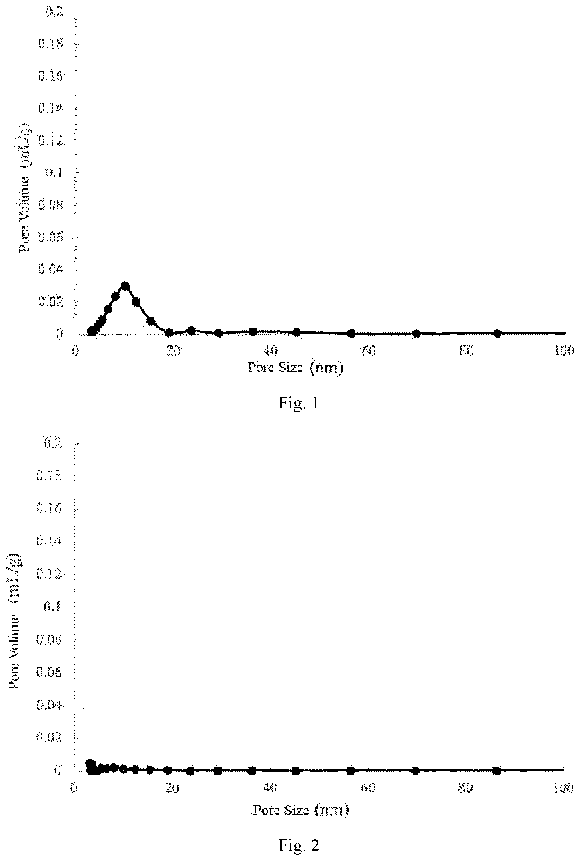

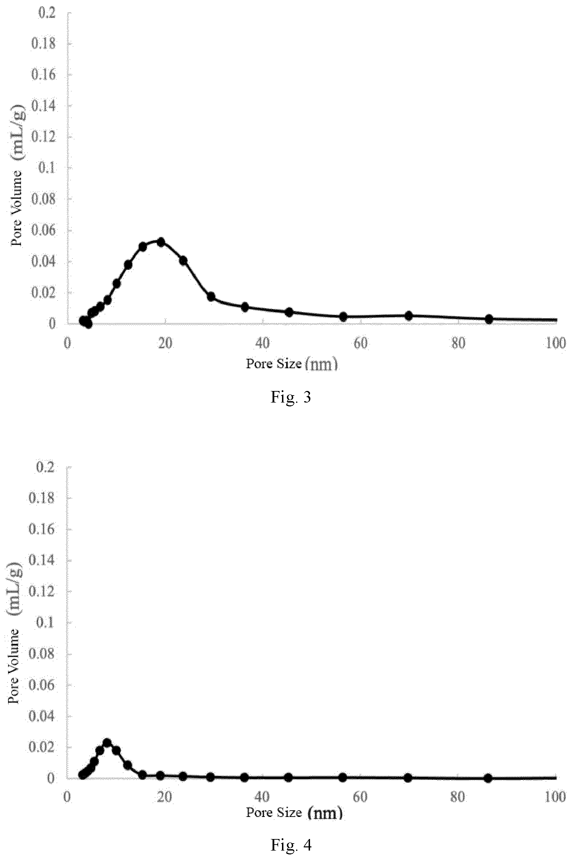

Figs. 1 to 5 showed the pore volume/pore size distribution curves for the microspheres of Examples 1 to 5 of the present disclosure.

### Example 10

### PK of Semaglutide Microspheres in Rats

Seven to eight weeks of healthy SD rats with weight of 200 to 250 g were used as study objects. The semaglutide microspheres samples were administered via subcutaneous injection in the dorsal cervical region of the rat. The dosages were all 18 mg/kg and about 0.2 mL of blood was drawn from the jugular vein at a specific time after administration. After blood collection, the blood collection tube containing the anticoagulant (K2EDTA) was repeatedly inverted several times to be thoroughly mixed. Centrifugation was carried out to obtain plasma samples. The plasma concentration (ng/mL) of semaglutide in the plasma samples at each time point was determined by LC-MS/MS method. The drug concentration versus time curves of the microspheres of Examples 1 to 5 were plotted, and the AUC_{inf} of the microspheres of Examples 1 to 5 was calculated.

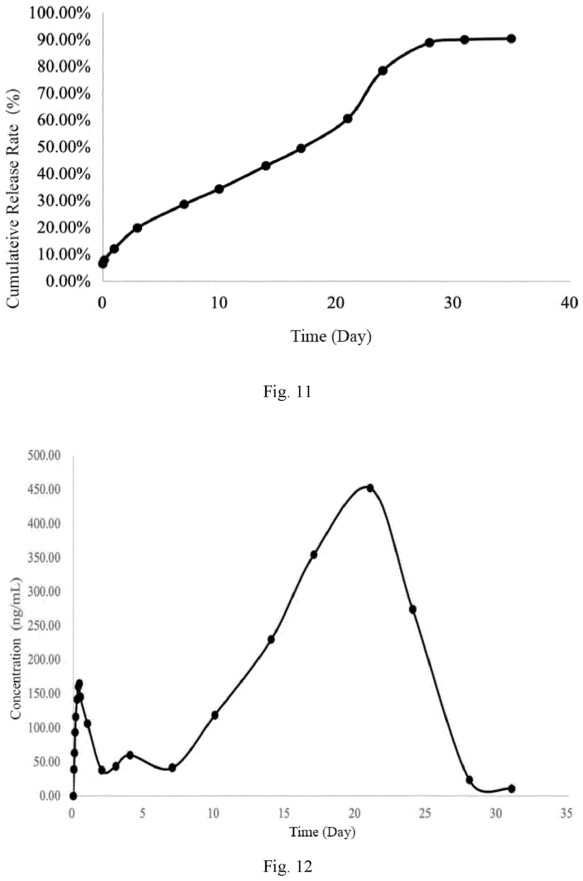

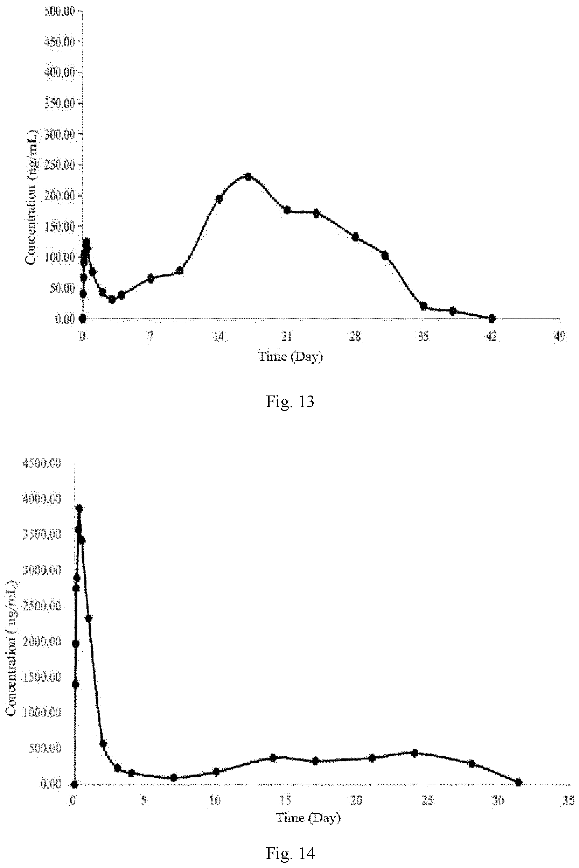

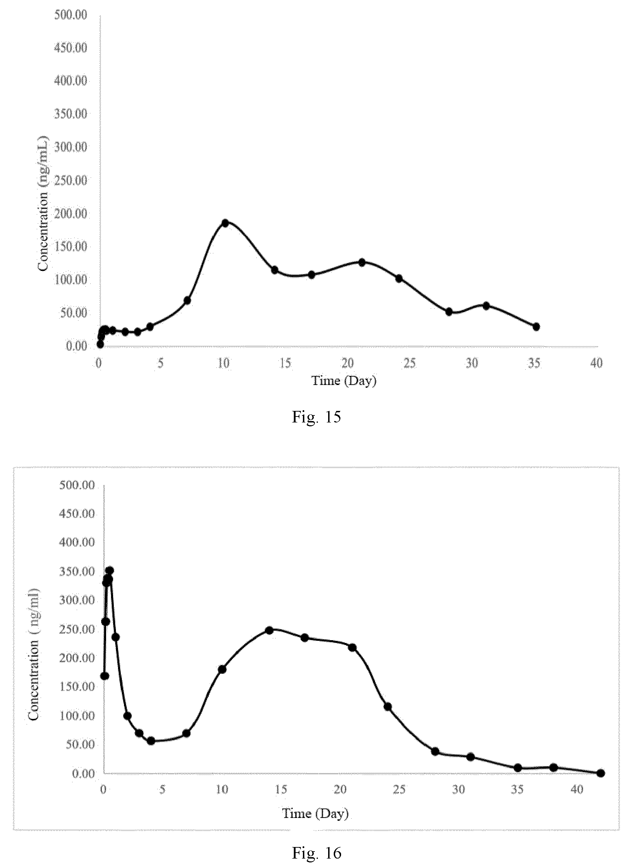

Figs. 11 to 15 showed the drug concentration versus time profiles of the microspheres of Examples 1 to 5 of the present disclosure.

| Examples | AUC_{inf} |
|---|---|
| Example 1 | 117610 |
| Example 2 | 112810 |
| Example 3 | 293176 |
| Example 4 | 78690 |
| Example 5 | 112030 |

In the present disclosure, relational terms, such as first and second and the like, are used solely to distinguish one entity or operation from another entity or operation without necessarily requiring or implying any such actual relationship or order between such entities or operations.

From the foregoing it will be appreciated that, although specific embodiments of the present disclosure have been described herein for illustrative purposes, various modifications or improvements may be made by those skilled in the art without departing from the spirit and scope of the present disclosure. Such variations or modifications are intended to fall within the scope of the claims appended hereto.

## Claims

1. Microspheres, comprising semaglutide and a pharmaceutically acceptable polymer, wherein drug loading of the microspheres is from 11.0% to 12.5% and 90% of cumulative pore volume of internal voids of the microspheres is from 0.013 to 0.15 mL/g.

2. Microspheres, comprising semaglutide and a pharmaceutically acceptable polymer, wherein drug loading of microspheres is from 11.0% to 20.0% and 90% of cumulative pore volume of internal voids of the microspheres is from 0.013 to 0.20 mL/g.

3. The microspheres of claim 1 or 2, further comprising sucrose, preferably in an amount of from 1% to 3% by weight, based on weight of the microspheres.

4. The microspheres of any one of claims 1 to 3, wherein the pharmaceutically acceptable polymer is poly(D,L-lactide-co-glycolide) polymer (PLGA), preferably in an amount of from 75% to 85% by weight based on weight of the microspheres.

5. The microspheres of any one of claims 1 to 4, wherein the pharmaceutically acceptable polymer is poly(D,L-lactide-co-glycolide) polymer (PLGA), preferably in an amount of from 80% to 85% by weight based on weight of the microspheres.

6. A pharmaceutical composition, comprising the microspheres of any one of claims 1 to 5.

7. A process for preparing microspheres, comprising:
providing a first aqueous solution comprising semaglutide;
providing an oil solution comprising a pharmaceutically acceptable polymer;
emulsifying the first aqueous solution and the oil solution to obtain a water-in-oil (W/O) emulsion;
mixing the water-in-oil (W/O) emulsion with a second aqueous solution to obtain a water-in-oil-in-water (W/O/W) emulsion; and
removing solvent in oil phase of the W/O/W emulsion to obtain the microspheres,
wherein drug loading amount of the microspheres is from 11.0% to 12.5% and 90% of cumulative pore volume of internal voids of the microspheres is from 0.013 to 0.15 mL/g.

8. A process for preparing microspheres, comprising:
providing a first aqueous solution comprising semaglutide;
providing an oil solution comprising a pharmaceutically acceptable polymer;
emulsifying the first aqueous solution and the oil solution to obtain a water-in-oil (W/O) emulsion;
mixing the water-in-oil (W/O) emulsion with a second aqueous solution to obtain a water-in-oil-in-water (W/O/W) emulsion; and
removing solvent in oil phase of the W/O/W emulsion to obtain the microspheres,
wherein drug loading amount of microspheres is from 11.0% to 20.0% and 90% of cumulative pore volume of internal voids of the microspheres is from 0.013 to 0.20 mL/g.

9. The process of claim 7 or 8, wherein the microspheres further comprise sucrose, preferably in an amount of from 1% to 3% by weight based on weight of the microspheres.

10. The process of any one of claims 7 to 9, wherein the pharmaceutically acceptable polymer is poly(D,L-lactide-co-glycolide) polymer (PLGA), preferably in an amount of from 75% to 85% by weight based on weight of the microspheres.

11. The process of any one of claims 7 to 10, wherein the pharmaceutically acceptable polymer is poly(D,L-lactide-co-glycolide) polymer (PLGA), preferably in an amount of from 80% to 85% by weight based on weight of the microspheres.

12. Microspheres prepared by the process of any one of claims 7 to 11.

13. Microspheres, comprising semaglutide and a pharmaceutically acceptable polymer, wherein the microspheres have drug loading of from 11.0% to 12.5% and 90% of cumulative pore volume of internal voids of the microspheres is from 0.013 to 0.15 mL/g, wherein the microspheres are prepared by a process comprising:
providing a first aqueous solution comprising semaglutide;
providing an oil solution comprising a pharmaceutically acceptable polymer;
emulsifying the first aqueous solution and the oil solution to obtain a water-in-oil (W/O) emulsion;
mixing the water-in-oil (W/O) emulsion with a second aqueous solution to obtain a water-in-oil-in-water (W/O/W) emulsion; and
removing solvent in oil phase of the W/O/W emulsion to obtain the microspheres.

14. Microspheres, comprising semaglutide and a pharmaceutically acceptable polymer, wherein the microspheres have drug loading of from 11.0% to 20.0% and 90% of cumulative pore volume of internal voids of the microspheres is from 0.013 to 0.20 mL/g, wherein the microspheres are prepared by a process comprising:
providing a first aqueous solution comprising semaglutide;
providing an oil solution comprising a pharmaceutically acceptable polymer;
emulsifying the first aqueous solution and the oil solution to obtain a water-in-oil (W/O) emulsion;
mixing the water-in-oil (W/O) emulsion with a second aqueous solution to obtain a water-in-oil-in-water (W/O/W) emulsion; and
removing solvent in oil phase of the W/O/W emulsion to obtain the microspheres.

15. The process of claim 13 or 14, wherein the microspheres further comprise sucrose, preferably in an amount of from 1% to 3% by weight based on weight of the microspheres.

16. The process of any one of claims 13 to 15, wherein the pharmaceutically acceptable polymer is poly(D,L-lactide-co-glycolide) polymer (PLGA), preferably in an amount of from 75% to 85% by weight based on weight of the microspheres.

17. The process of any one of claims 13 to 16, wherein the pharmaceutically acceptable polymer is poly(D,L-lactide-co-glycolide) polymer (PLGA), preferably in an amount of from 80% to 85% by weight based on weight of the microspheres.

18. A method for treating diabetes, comprising administering to a subject in need thereof a therapeutically effective amount of the microspheres of any one of claims 1 to 6 and 13 to 17.

19. A method for reducing body weight, comprising administering to a subject in need thereof a therapeutically effective amount of the microspheres of any one of claims 1 to 6 and 13 to 17.
